Europäisches Patentamt

European Patent Office

Office européen des brevets

(11) Veröffentlichungsnummer: **0 306 543 B1**

(19)

# EUROPÄISCHE PATENTSCHRIFT

(45) Veröffentlichungstag der Patentschrift: **04.12.91**

(51) Int. Cl.⁵: **A61F 6/14**

(21) Anmeldenummer: **87113129.8**

(22) Anmeldetag: **08.09.87**

(54) Empfängnisverhütende Intrauterinvorrichtung.

(43) Veröffentlichungstag der Anmeldung:
**15.03.89 Patentblatt 89/11**

(45) Bekanntmachung des Hinweises auf die
Patenterteilung:
**04.12.91 Patentblatt 91/49**

(84) Benannte Vertragsstaaten:
**CH DE FR GB IT LI**

(56) Entgegenhaltungen:
**EP-A- 0 147 274**
**EP-A- 0 179 518**
**CH-A- 585 550**
**DE-A- 3 209 290**
**US-A- 4 249 531**

(73) Patentinhaber: **NAUCHNO-PROIZVODSTVENNOE OBIEDINENIE MEDIN-STRUMENT**
**ulitsu K. Tinchurina, 31**
**Kazan(SU)**

(72) Erfinder: **Gainutdinova, Raisa Vladimirovna**
**ulitsa Spartakovskaya, 80, kv. 15**
**Kazan(SU)**
Erfinder: **Jurov, Boris Andreevich**
**ulitsa Kurchatova, 6, kv.99**
**Kazan(SU)**
Erfinder: **Mazo, Bentsian Moiseevich**
**ulitsa Gagarina, 73, kv. 51**
**Kazan(SU)**
Erfinder: **Petrova-Pokrovskaya, Vera Mitrofanovna**
**ulitsa Frunze, 13a, kv. 2**
**Kazan(SU)**

(74) Vertreter: **Finck, Dieter et al**
**Patentanwälte v. Füner, Ebbinghaus, Finck**
**Mariahilfplatz 2 & 3**
**W-8000 München 90(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft eine empfängnisverhütende Intrauterinvorrichtung mit einem Streifen, mit einem mit dem Streifen verbundenen und mit ihm ein T-förmiges Element bildenden Stab, mit einer auf den Stab aufgesetzten Schraubenwendel und mit einem mit dem Stab verbundenen Faden zur jederzeit möglichen Feststellung, ob sich eine empfängnisverhütende Intrauterinvorrichtung innerhalb der Gebärmutterhöhle befindet.

Aus der US-A-41 98 966 ist eine empfängnisverhütende Intrauterinvorrichtung bekannt, die einen Streifen, einen mit dem Streifen verbundenen und mit ihm ein T-förmiges Element bildenden Stab, der mit einer Kupferwendel versehen ist, und einen an dem Stab befestigten Faden zur dynamischen Überwachung der Intrauterinvorrichtung vorsieht.

Die Wendel besteht aus chemisch reinem Kupfer, wodurch das Gewicht der ganzen empfängnisverhütenden Intrauterinvorrichtung groß ist und die Gefahr der Expulsion besteht.

Die Kupferwendel löst sich im Laufe der Gebrauchszeit allmählich auf und reißt schließlich unter Bildung von scharfen, hervortretenden Enden, die die Gebärmutterwand verletzen und Blutungen hervorrufen können.

Bekannt ist ferner eine empfängnisverhütende Intrauterinvorrichtung (Nova T Cu 200 Ag, AG Khukhtamyaki, Leiras, Finnland), die einen Streifen, einen mit dem Streifen verbundenen und mit ihm ein T-förmiges Element bildenden Stab, eine an dem Stab angeordnete Wendel aus Kupferdraht mit einer Silberunterlage und einen am Ende des Stabes befestigten Faden aufweist, der jederzeit die Feststellung ermöglicht, ob eine empfängnisverhütende Intrauterinvorrichtung innerhalb der Gebärmutterhöhle vorhanden ist.

Durch die aus Kupferdraht mit Silberunterlage bestehende Wendel ist das Gewicht der ganzen Intrauterinvorrichtung hoch, so daß die Gefahr der Expulsion besteht.

Der Erfindung liegt die Aufgabe zugrunde, eine empfängnisverhütende Intrauterinvorrichtung zu schaffen, deren Expulsion im Laufe der Gebrauchszeit im wesentlichen aus geschlossen werden kann.

Diese Aufgabe wird erfindungsgemäß dadurch gelöst, daß die Schraubenwendel zumindest zwei Schichten aufweist, wobei die erste Schicht aus einem elastischen Polymerstoff besteht, die Rolle einer Unterlage übernimmt und dem Stab unmittelbar zugewandt ist, während die zweite Schicht aus einem Metall besteht.

Zweckmäßigerweise weist die Schraubenwendel eine dritte Schicht aus Metall auf, die auf der ersten Schicht liegt und dem Stab zugewandt ist.

Vorteilhafterweise ist die Schraubenwendel mit zwei aus Metall bestehenden und auf die erste Schicht gelegten Flankenschichten versehen.

Vorzugsweise ist die die erste Schicht bildende Unterlage hohl, um eine komplexe Kontrazeption zu ermöglichen.

Dadurch, daß die Unterlage in allen Ausführungsvarianten aus einem elastischen Polymerstoff besteht, ist die Intrauterinvorrichtung sehr leicht. Außerdem kann die Schraubenwendel selbst bei völliger Auflösung der Metallschichten nicht reißen, d.h. es wird eine atraumatische Wirkung gesichert.

Anhand von Zeichnungen werden Ausführungsbeispiele der Erfindung näher erläutert. Es zeigt:

Fig. 1        die empfängnisverhütende Intrauterinvorrichtung in einer teilweise geschnittenen Seitenansicht und

Fig. 2 bis 6    im Schnitt verschiedene Ausführungsvarianten der Schraubenwendel.

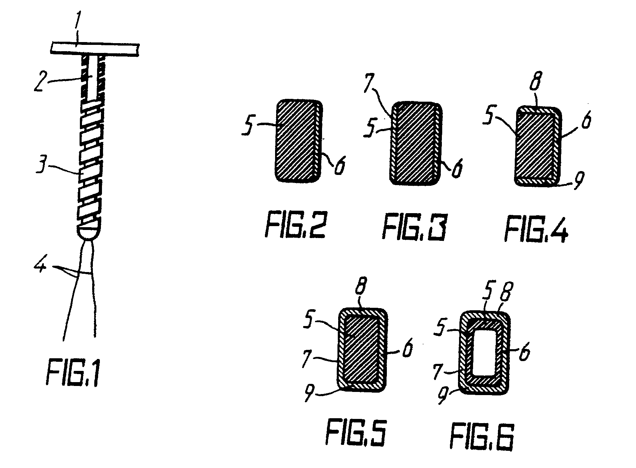

Die in Fig. 1 gezeigte empfängnisverhütende Intrauterinvorrichtung hat einen Streifen 1, einen mit ihm verbundenen und mit ihm ein T-förmiges Element bildenden Stab 2, eine auf dem Stab 2 aufgesetzte Schraubenwendel 3 und einen Faden 4, der jederzeit die Feststellung ermöglicht, ob sich eine empfängnisverhütende Intrauterinvorrichtung innerhalb der Gebärmutterhöhle befindet.

Die in Fig. 2 gezeigte Schraubenwendel 3 hat zwei Schichten 5 und 6. Die erste Schicht 5 besteht aus einem elastischen Polymerstoff, der eine Unterlage bildet. Die erste Schicht 5 ist dem Stab 2 (Fig. 1) zugewandt. Die zweite Schicht 6 besteht aus einem Metall.

Die Schraubenwendel 3 kann eine dritte Schicht 7 (Fig. 3) aus Metall aufweisen, die auf die erste Schicht 5 gelegt und dem Stab 2 (Fig. 1) zugewandt ist.

Die Schraubenwendel 3 kann zwei Flankenschichten 8, 9 (Fig. 4, 5) besitzen, von denen jede aus Metall besteht und die jeweils auf der ersten Schicht 5 angeordnet sind.

Die erste, als Unterlage dienende Schicht 5 (Fig. 6) kann hohl ausgeführt werden, um eine komplexe Kontrazeption zu ermöglichen. Bei den erwähnten Ausführungsvarianten bestehen die auf die Unterlage aufgetragenen Metallschichten beispielsweise aus Kupfer, Silber oder Nickel.

Die empfängnisverhütende Intrauterinvorrichtung wirkt wie folgt:
Die zu einer Linie durch Zusammenklappen des Streifens 1, des Stabes 2 mit der Schraubenwendel 3 und des Fadens 4 koaxial zusammengelegte empfängnisverhütende Intrauterinvorrichtung wird in einer nicht gezeigten Sonde angeordnet und mit deren Hilfe in die Gebärmutterhöhle eingeführt, wo sie eine der Gestalt der Gebärmutterhöhle entsprechende Stellung einnimmt. Innerhalb der Gebärmutterhöhle wird die Intrauterinvorrichtung durch den Streifen 1 an die Wand der Gebärmutter angedrückt, während der Faden mit seinem freien Ende aus dem Gebärmutterhals heraushängt.

Während des Gebrauchs der empfängnisverhütenden Intrauterinvorrichtung lösen sich die Metallschichten der Schraubenwendel 3 allmählich auf. Selbst bei vollkommener Auflösung der Metallschichten zerreißt die aus einem elastischen Polymerstoff hergestellte Unterlage nicht, wodurch eine Perforation der Gebärmutterwände und damit verbundene Blutungen vermieden werden. Durch Benutzung einer Schraubenwendel mit der elastischen Unterlage und mit den auf diese aufgetragenen Metallschichten ist die Intrauterinvorrichtung sehr leicht, was die Gefahr von Expulsionen verringert.

## Patentansprüche

1. Empfängnisverhütende Intrauterinvorrichtung mit einem Streifen (1), mit einem mit dem Streifen (1) verbundenen und mit ihm ein T-förmiges Element aus Künststoff bildenden Stab (2), mit einer auf den Stab (2) aufgesetzten Metall aüfweitenden Schraubenwendel (3) und mit einem mit dem Stab (2) verbundenen Faden (4) zur jederzeit möglichen Feststellung, ob sich eine empfängnisverhütende Intrauterinvorrichtung innerhalb der Gebärmutterhöhle befindet, dadurch **gekennzeichnet,** daß die Schraubenwendel (3) zumindest aus zwei Schichten (5,6) aufgebaut ist, von denen die erste Schicht (5) aus einem elastischen Polymerstoff eine Unterlage bildet und dem Stab (2) unmittelbar zugewandt ist, während die zweite Schicht (6) aus einem Metall besteht.

2. Empfängnisverhütende Intrauterinvorrichtung nach Anspruch 1, dadurch **gekennzeichnet,** daß die Schraubenwendel (3) eine aus Metall bestehende dritte Schicht (7) aufweist, die über die erste Schicht (5) gelegt und dem Stab (2) zugewandt ist.

3. Empfängnisverhütende Intrauterinvorrichtung nach Anspruch 1, dadurch **gekennzeichnet,** daß die Schraubenwendel (3) zwei Flankenschichten (8, 9) aufweist, von denen jede aus Metall besteht und auf die erste Schicht (5) gelegt ist.

4. Empfängnisverhütende Intrauterinvorrichtung nach Anspruch 3, dadurch **gekennzeichnet,** daß die eine Unterlage bildende erste Schicht (5) zur Durchführung einer komplexen Kontrazeption hohl ausgeführt ist.

## Claims

1. A contraceptive intra-uterine device with a strip (1), with a bar (2), which is connected to the strip (1) and together with the strip forms a T-shaped element made of synthetic material, with a screw spiral (3), which is mounted on the bar (2) and has metal, and with a thread (4), which is connected to the bar (2) and makes it possible at any time to ascertain whether a contraceptive intra-uterine device is present within the uterine cavity, characterised in that the screw spiral (3) is at least constructed from two layers (5, 6), of which the first layer (5), which is made of an elastic polymer material, forms a base and directly faces the bar (2), whilst the second layer (6) consists of a metal.

2. A contraceptive intra-uterine device according to Claim 1, characterised in that the screw spiral (3) has a third layer (7), which consists of metal, is placed over the first layer (5) and faces the bar (2).

3. A contraceptive intra-uterine device according to Claim 1, characterised in that the screw spiral (3) has two flank layers (8, 9), each of which consists of metal and is placed on the first layer (5).

4. A contraceptive intra-uterine device according to Claim 3, characterised in that the first layer (5), which forms a base, is designed so as to be hollow in order to effect a complex contraception.

## Revendications

1. Dispositif contraceptif intra-utérin avec une bande (1) avec une tige (2) reliée à la bande (1) et formant avec elle un élément en forme de T en matière synthétique, avec une spirale (3) usinée en métal et placée sur la tige (2) et avec un fil (4) relié à la tige (2) pour permettre de savoir en tout moment si un dispositif contraceptif intra-utérin se trouve à l'intérieur de la cavité utérine, caractérisé en ce que la spirale (3) est formée d'au moins deux couches (5, 6) dont la première couche (5) forme une base en un polymère élastique et est tournée directement vers la tige (2) tandis que la seconde couche (6) se compose d'un métal.

2. Dispositif contraceptif intra-utérin selon la revendication 1, caractérisé en ce que la spirale (3) présente une troisième couche (7) se composant d'un métal, qui est placée sur la première couche (5) et est tournée vers la tige (2).

3. Dispositif contraceptif intra-utérin selon la revendication 1, caractérisé en ce que la spirale (3) présente deux couches latérales (8, 9) dont chacune est en métal et est placée sur la première couche (5).

4. Dispositif contraceptif intra-utérin selon la revendication 3, caractérisé en ce que la première couche (5) formant une base est réalisée creuse pour l'accomplissement d'une contraception complexe.

FIG.1

FIG.2

FIG.3

FIG.4

FIG.5

FIG.6